Europäisches Patentamt

**European Patent Office**

Office européen des brevets

(11) Veröffentlichungsnummer: **0 268 177**
**B1**

(12) **EUROPÄISCHE PATENTSCHRIFT**

(45) Veröffentlichungstag der Patentschrift:
12.09.90

(21) Anmeldenummer: 87116508.0

(22) Anmeldetag: 09.11.87

(51) Int. Cl.⁵: **A01N 63/04, C12N 1/04**

(54) Schädlingsbekämpfungs- und Pflanzenbehandlungsmittel.

(30) Priorität: 20.11.86 DE 3639504

(43) Veröffentlichungstag der Anmeldung:
25.05.88 Patentblatt 88/21

(45) Bekanntmachung des Hinweises auf die Patenterteilung:
12.09.90 Patentblatt 90/37

(84) Benannte Vertragsstaaten:
BE CH DE FR GB IT LI NL SE

(56) Entgegenhaltungen:
EP-A- 102 702
EP-A- 0 134 873
EP-A- 0 145 197
EP-A- 0 159 891
DE-A- 2 611 801
DE-A- 2 617 892

COMMONWEALTH AGRICULTURAL BUREAU, 1983,
Zusammenfassung 83890397, Slough, GB; R.A. DAOUST
et al.: "Effect of formulation on the virulence of
metarhizium anisopliae conidia against mosquito
larvae"
COMMONWEALTH AGRICULTURAL BUREAU, 1983,
Zusammenfassung 71197346, Slough, GB; R.A. DAOUST
et al.: "Effect of formulation on the viability of
metarhizium anisopliae conidia"

(73) Patentinhaber: BAYER AG,
D-5090 Leverkusen 1 Bayerwerk(DE)

(72) Erfinder: Andersch, Wolfram, Dr., Bodinusstrasse 6,
D-5000 Köln(DE)
Erfinder: Hartwig, Jürgen, Dr., Franz-Esser-Strasse 44,
D-5090 Leverkusen 3(DE)
Erfinder: Homeyer, Bernhard, Dr., Obere Strasse 28,
D-5090 Leverkusen 3(DE)
Erfinder: Stenzel, Klaus, Dr.,
Rudolf-Breitscheld-Strasse 3, D-4000 Düsseldorf 13(DE)

(56) Entgegenhaltungen: (Fortsetzung)
**Die Akte enthält technische Angaben, die nach dem
Eingang der Anmeldung eingereicht wurden und die
nicht in dieser Patentschrift enthalten sind.**

## Beschreibung

Die vorliegende Erfindung betrifft neue Schädlingsbekämpfungsmittel und Pflanzenbehandlungsmittel, welche aus trägerfreien Zellgranulaten von Mikroorganismen bestehen oder wenigstens ein trägerfreies Zellgranulat von Mikroorganismen enthalten, ein Verfahren zu ihrer Herstellung und ihre Verwendung sowie neue Mikroorganismenstämme.

Es ist bereits bekannt geworden, daß bestimmte Mikroorganismen (Bakterien, Pilze und Viren) gegenüber Schädlingen, wie Insekten oder Nematoden pathogen sein können und in der Schädlingsbekämpfung verwendet werden können. Die Bereitstellung geeigneter Mikroorganismen-Zubereitungen (Formulierungen) mit einer standardisierten Wirksamkeit stößt jedoch in vielen Fällen auf große Schwierigkeiten, weil die Formulierung der Mikroorganismen häufig ihre Wirksamkeit und Lagerfähigkeit nachteilig beeinflußt.

Es wurden nun die neuen Schädlingsbekämpfungsmittel und Pflanzenbehandlungsmittel gefunden, welche dadurch gekennzeichnet sind, daß sie aus trägerfreien Zellgranulaten von Mikroorganismen, die zur Schädlingsbekämpfung oder Pflanzenbehandlung geeignet sind, bestehen oder wenigstens ein trägerfreies Zellgranulat von solchen Mikroorganismen enthalten, wobei die Zellgranulate aus Pilzen oder Bakterien gebildet werden, die zur Myzelbildung fähig sind und wobei die Zellgranulate eine im wesentlichen kugelige Gestalt haben und Durchmesser von 0,05 bis 2 mm aufweisen.

Unter Schädlingsbekämpfungsmittel sollen alle erfindungsgemäßen Mittel verstanden werden, welche zur Bekämpfung von unerwünschten tierischen und pflanzlichen Schädlingen und Lästlingen (wie schädliche Arthropoden und Nematoden, Unkräuter und Ungräser, schädliche Bakterien und Pilze) verwendet werden können. Im allgemeinen beruht die Wirksamkeit dieser Schädlingsbekämpfungsmittel auf der antagonistischen Fähigkeit (Parasitisierung, Toxinbildung, Konkurrenzverhalten) der verwendeten Mikroorganismen gegenüber den Schädlingen, welche zu ihrer Eindämmung oder Abtötung führt. Die Schädlingsbekämpfungsmittel werden vorzugsweise in den Bereichen Landwirtschaft, Forst, Gartenbau, Haushalt und Hygiene, Vorratsschutz und Materialschutz, insbesondere zum Schutz von Pflanzen oder Ernteprodukten eingesetzt. Bevorzugte erfindungsgemäße Schädlingsbekämpfungsmittel sind solche, die zur Bekämpfung von Schädlingen verwendet werden können, die im Bodenbereich vorkommen können.

Unter Pflanzenbehandlungsmittel sollen alle erfindungsgemäßen Mittel verstanden werden, welche dazu geeignet sind, das Wachstum von Pflanzen zu beeinflussen bzw. zu regulieren (wie durch die Ausscheidung von Pflanzenhormonen, die Bereitstellung von Nährstoffen usw.). Sie können insbesondere in den Gebieten Landwirtschaft, Forst und Gartenbau eingesetzt werden.

Bevorzugte erfindungsgemäße Mittel sind die Schädlingsbekämpfungsmittel, vorzugsweise zur Bekämpfung von tierischen Schädlinen (vorzugsweise Arthropoden und Nematoden, insbesondere Insekten und Nematoden, ganz besonders bevorzugt von Insekten) und von mikrobiellen Schädlingen (wie schädlichen Bakterien und Pilzen), insbesondere von tierischen Schädlingen.

Die erfindungsgemäß verwendeten trägerfreien Zellgranulate von Mikroorganismen sind im wesentlichen kugelförmige Gebilde, welche aus gewebeartig verwachsenen Mikroorganismenzellen zusammengesetzt sind und keine Trägermaterialien enthalten. Sie sind mechanisch so stabil, daß sie sich bei der Herstellung, Aufarbeitung, Abfüllung und Anwendung nicht in unverwünschter Weise z.B. durch Abrieb nachteilig verändern. Die Zellgranulate weisen Durchmesser von 0,05 bis 2,0 mm, vorzugsweise von 0,1 bis 1,5 mm und besonders bevorzugt von 0,5 bis 1,0 mm auf.

Als erfindungsgemäß in Form der Zellgranulate einsetzbare Mikroorganismen kommen alle Mikroorganismen (Bakterien und Pilze) in Frage, welche über die Fähigkeit zur Myzelbildung verfügen. Sie müssen weiterhin (unter den Bedingungen des erfindungsgemäßen Vakuums) Zellaggregationen und Zellgranulate bilden können. Wenn die erfindungsgemäßen Zellgranulate zur Schädlingsbekämpfung bestimmt sind, müssen die Mikroorganismen fähig sein, die zu bekämpfenden Schädlinge so in ihrer Lebenskraft oder in ihrer Vermehrungsfähigkeit zu beeinträchtigen, daß sie aufgrund der Einwirkung durch die Schädlingsbekämpfungsmittel ausreichend kontrolliert werden können. Hierzu müssen die erfindungsgemäß verwendbaren Mikroorganismen in der Lage sein, Stoffe in die Umgebung abzugeben, welche auf die Schädlinge entsprechend einwirken oder aber fähig sein die Schädlinge ausreichend zu parasitisieren.

Wenn die erfindungsgemäß einzusetzenden Zellgranulate als Pflanzenbehandlungsmittel verwendet werden sollen, müssen die Mikroorganismen fähig sein, Stoffe in die Umgebung abzugeben, welche auf die Pflanzen einwirken, z.B. Phytohormone oder Nährstoffe oder welche die Pflanzen negativ beeinflussenden Stoffe unschädlich machen.

Die in den Zellgranulaten verwendeten Mikroorganismen sollen keine pathogenen Eigenschaften gegenüber Warmblütern aufweisen und darüber hinaus keine Nützlinge (z.B. Regenwürmer, Bienen) schädigen.

Zur Bildung von Zellgranulaten ist eine Vielzahl von Mikroorganismen, vorzugsweise von Pilzarten aus den taxonomischen Klassen der Phycomyceten, Ascomyceten, z. B. Chaetomium, Basidiomyceten und den Deuteromyceten befähigt, insbesondere die Vertreter der Fungi imperfecti, wie z.B. verschiedene Arten von Aspergillus, Alternartia, Aphanocladium, Beauveria, Coniothyrium, Colletotrichum, Meria (Drechmeria), Penicillium, Fusarium, Gliocladium, Pseudocercosporella, Trichoderma, Verticillium, Paecilomyces, insbesondere auch von Metarhizium und Gliocladium, besonders bevorzugt von Metarhizium. Zahlreiche Stämme dieser Pilze weisen eine antagonistische Aktivität gegen bodenbürtige, pflanzenpathogene Pilze, wie z.B. Trichoderma hamatum und Gliocladium roseum, gegen Unkräuter, wie z.B. Alternaria cassiae, Fusarium lateritum, Fusarium solani, bzw. gegen Schadinsekten, wie z.B. Verticillium lecanii, Aspergillus parasiticus, insbesondere aber Metarhizium anisopliae auf.

Unter den Mikroorganismen werden fungizide, nematopathogene und entomophathogene Mikroorganismen (insbesondere Pilze der Klasse Deuteromycetes) bevorzugt. Besonders bevorzugt werden nematophatogene und entomophatogene Mikroorganismen.

Ganz besonders bevorzugt werden Pilze der Gattung Metarhizium, insbesondere der Art Metarhizium anisopliae und hierbei besonders die Metarhizium anisopliae Stämme P 0001 und P 0003, insbesondere der Stamm P 0001. Diese neuen Stämme, welche erfindungsgemäß besonders günstig verwendbar und Gegenstand der vorliegenden Erfindung sind, wurden bei der Deutschen Sammlung von Mikroorganismen (DSM), Grisebachstraße 8, D-3400 Götringen, Bundesrepublik Deutschland in Übereinstimmung mit den Bestimmungen des Budapester Vertrages über die internationale Anerkennung der Hinterlegung von Mikroorganismen für die Zwecke von Patentverfahren am 24.10.1986 hinterlegt und haben die Hinterlegungsnummern:

DSM 3884 (P 0001) und
DMS 3885 (P 0003).

Die vorliegende Erfindung erstreckt sich auch auf die Mutanten und Varianten dieser Stämme, welche die für die Ausführung der Erfindung wesentlichen Merkmale und Eigenschaften aufweisen. Diese Metarhizium Stämme ergeben nach dem erfindungsgemäßen Verfahren Zellgranulate mit sehr günstigen physikalischen und biologischen Eigenschaften, welche die Verwendung dieser Zellgranulate als Schädlingsbekämpfungsmittel ermöglichen, vorzugsweise zur Bekämpfung von Arthropoden und Nematoden, insbesondere von Insekten und Nematoden (besonders von Insekten) und ganz besonders bevorzugt von Bodeninsekten, also Insekten, welche im Boden, auf dem Boden oder am Pflanzenmaterial in der Nähe des Bodens vorkommen.

Die vorliegende Erfindung betrifft auch die neue Verwendung von den oben aufgeführten Mikroorganismen, welche zur Myzelbildung fähig sind, zur Erzeugung der neuen Schädlingsbekämpfungsmittel und Pflanzenbehandlungsmittel.

Die neuen trägerfreien Zellgranulate können Nährstoffe enthalten (z.B. solche die beim erfindunggemäßen Herstellungsverfahen bei der Fermentation verwendet werden). Diese Nährstoffe können ein rasches Wachstum der Mikroorganismen nach der Anwendung der neuen Mittel begünstigen.

Sie können auch protektiv wirkende Substanzen enthalten, die eine zu starke Austrocknung der Mikroorganismenzellen verhindern (z.B. Polyalkohole, wie Zucker oder Glycerin).

Zur Verbesserung der Lagerfähigkeit können die neuen Zellgranulate untoxische, antioxidative Stoffe (wie Ascorbinsäure, 2,3-tert.-Butyl-4-hydroxi-anisol, 2,6-Di-tert.-butyl-p-kresol, Propylgallate oder Nordihydroguaiaretsäure) enthalten.

Eine rasche Rehydratation nach der Anwendung kann dadurch erreicht werden, daß die neuen Zellgranulate hygroskopisch wirkende Stoffe (wie geeignete Polyalkohole, z.B. Glycerin, Zucker, Oligo- und Polysaccharide und ihre Derivate.

In einer speziellen Ausführungsform weisen die erfindungsgemäßen Zellgranulate im wesentlichen an der Oberfläche eine erhöhte Menge an Mikroorganismen-Dauerstadien (z. B. ruhende oder schlafende Stadien wie Sporen oder Konidien) auf. Im allgemeinen ergibt sich hieraus eine besonders langandauernde Lagerfähigkeit bei einer raschen Vermehrung und Ausbreitung der in Form der Zellgranulate applizierten Mikroorganismen.

In einer besonders bevorzugten Ausführungsform enthalten die erfindungsgemäßen Zellgranulate jedoch keinerlei der oben angeführten Additive. Bevorzugt werden erfindungsgemäße Zellgranulate, welche keine erhöhte Menge an Dauerstadien haben.

Die erfindungsgemäßen Mittel können neben den trägerfreien Zellgranulaten weitere Schädlingsbekämpfungsmittel (z.B. Fungizide, Insektizide oder Herbizide) oder Pflanzenbehandlungsmittel (z.B. Düngsmittel) als Zumischungen enthalten.

Bevorzugt werden erfindungsgemäße Mittel, welche aus trägerfreien Zellgranulaten (ohne weitere Beimischung) bestehen.

Gegenüber herkömmlichen Mikroorganismen-Zubereitungen, welche als Schädlingsbekämpfungs- oder Pflanzenbehandlungsmittel vorgeschlagen oder verwendet werden, weisen die neuen erfindungsgemäßen Zellgranulate aufgrund ihrer physikalischen und biologischen Eigenschaften wesentliche Vorteile auf.

Die Zellgranulate sind nach dem erfindungsgemäßen Verfahren leicht herstellbar. Sie können im Produktionsverfahren besonders einfach abgetrennt werden. Ihre Handhabbarkeit bei der Bearbeitung (Abtrennung, Trocknung, Abfüllung, Lagerung) und Anwendung ist gut, da sie nicht stauben, eine definierte Korngröße und sehr gute Schütteigenschaften aufweisen sowie sehr leicht dosiert und einfach appliziert werden können. Außer einer guten mechanischen Stabilität weisen die neuen Zellgranulate eine hohe Lagerstabilität auf, so daß sie ihre volle biologische Wirkung auch noch nach längerer Lagerung entfalten und somit der bei der Produktion vorgegebene Wirkungsstandard erhalten bleibt, was bei Mitteln zur Schädlingsbekämpfung mit biologischen Materialien für die Praxis von einer besonderen Bedeutung ist.

Weiterhin wurde gefunden, daß man die neuen Schädlingsbekämpfungsmittel und Pflanzenbehandlungsmittel, dadurch erhält, daß man
(A) zur Initiation der Zellaggregation (erster Verfahrensschritt)

a) im Falle von Mikroorganismenzellen mit einer im wesentlichen hydrophoben Zelloberfläche, nach Zugabe eines oder mehrerer Detergentien zu einer wäßrigen Aufschlämmung der in einer Vorkultur erhaltenen Mikroorganismen, die zur Schädlingsbekämpfung oder Pflanzenbehandlung geeignet sind, die Zellen suspendiert und die Zellsuspension in Wasser oder ein wäßriges Nährmedium einträgt, so daß eine Zellaggregation erfolgt oder

b) im Falle von Mikroorganismenzellen ohne eine im wesentlichen hydrophobe Zelloberfläche durch die Zugabe von Säuren oder Basen zu einer Aufschlämmung der in einer Vorkultur erhaltenen Mikroorganismen, die zur Schädlingsbekämpfung oder Pflanzenbehandlung geeignet sind, in Wasser oder einem wäßrigen Nährmedium den pH-Wert so einstellt, daß eine Zellaggregation erfolgt oder

c) eine Aufschlämmung bzw. Suspension der in einer Vorkultur erhaltenen Mikroorganismen, die zur Schädlingsbekämpfung oder Pflanzenbehandlung geeignet sind, in Wasser oder einem Nährmedium mit Flocculantien versetzt, so daß eine Zellaggregation erfolgt und
anschließend

(B) zur Bildung der Zellgranulate (zweiter Verfahrensschritt) die erhaltenen Zellaggregationen in einem Nährmedium, welches gegebenenfalls komplexbildende Substanzen enthält, unter aeroben Bedingungen einer Fermentation unterwirft und die gebildeten Zellgranulate abtrennt und

(C) gegebenenfalls zur Bildung einer im wesentlichen auf der Oberfläche der Zellgranulate erhöhten Menge von Mikroorganismen-Dauerstadien, die abgetrennten Zellgranulate einer Inkubation unter den Bedingungen einer Oberflächenkultur unterwirft und

(D) die erhaltenen Zellgranulate, gegebenenfalls nach dem Hinzufügen von bzw. der Behandlung mit Nährstoffen, protektiv wirkenden Substanzen, antioxidativ wirkenden Stoffen und/oder die Rehydratation unterstützenden Stoffen, trocknet und gegebenenfalls mit weiteren Schädlingsbekämpfungsmitteln oder Pflanzenbehandlungsmitteln mischt.

Das erfindungsgemäße Verfahren zur Herstellung der Zellgranulate besteht im wesentlichen aus zwei Phasen. In der ersten Phase wird eine Zellaggregation initiiert, wobei die besonderen biochemischen Eigenschaften der jeweiligen Mikroorganismenzellen genutzt werden, und in der zweiten Phase werden die eigentlichen Zellgranulate in einem Fermentationsprozeß gebildet.

Zunächst wird eine Vorkultur (Inokulum) der Mikroorganismen nach dem üblichen Methoden der Oberflächenkulturen oder Flüssigkulturen, z.B. als Schrägröhrchen, auf Nähragarplatten, auf als Nährsubstrat verwertbaren Trägermaterialien oder in Schüttelkolben mit flüssigen Medien hergestellt. Hierbei können z.B. die bei der Fermentation unten beschriebenen Nährmedien verwendet werden.

Die Initiation der Zellaggregation kann nach verschiedenen Methoden erfolgen. Die jeweils geeignete Methode kann mit Hilfe einfacher Reihenversuche leicht ermittelt werden.

So können zu einer Aufschlämmung der mikroorganismen in Wasser oder eine wäßrigen Nährlösung anorganische oder organische Flocculantien, z.B. Quelltone, wie Bentonit, Montmorillonit und Attapulgit oder Stärke, Leim, Polyacrylamid, Carboxymethylcellulose und Polyethylenoxid hinzugefügt werden um eine Zellaggregation zu erreichen.

Im Falle von Mikroorganismenzellen mit einer im wesentlichen hydrophoben Zelloberfläche, vorzugsweise bei Dauerstadien der Mikroorganismen (Sporen oder Konidien) wird zu einer Aufschlämmung der Zellen in Wasser oder einer Nährstofflösung ein Detergens (oder eine Mischung von Detergentien) hinzugefügt, z.B. Polyoxyethylenderivate von Sorbitanhydriden (wie Tween 80) und die Zellen werden suspendiert. Vorzugsweise wird eine Detergens-Konzentration von 0,01 bis 5,0 % (w/v), insbesondere von 0,1 bis 1,0 % (w/v) erzeugt. Die Suspension enthält vorzugsweise $10^3$ bis $10^9$ Zellen/ml, insbesondere $10^5$ bis $10^7$ Zellen/ml. Die Zellsuspension wird in ein wäßriges flüssiges Nährmedium, vorzugsweise durch Zuspritzen, eingetragen. Vorzugsweise beträgt hierbei das Volumen von Zellsuspension und Nährmedium ein Verhältnis von mindestens 1:5, insbesondere von 1:50 bis 1:100. Durch die Ausdünnung des Detergens kommt es zu der gewünschten Aggregation der Mikroorganismenzellen.

Im Falle von Mikroorganismen ohne eine im wesentlichen hydrophobe Zelloberfläche, vorzugsweise bei vegetativen Zellen, kann eine Aggregation der Zellen erzielt werden, wenn man in einer Aufschlämmung oder Suspension der Zellen in Wasser oder einem wäßrigen Nährmedium durch eine entsprechende Einstellung des pH-Wertes die positiv- und negativ geladenen Molekülgruppen an der Zelloberfläche neutralisiert bis die gewünschte Zellaggregation auftritt. Die pH-Wert-Einstellung kann durch die Zugabe von organischen oder anorganischen Säuren oder Basen erreicht werden (z.B. Schwefelsäure, Salzsäure, Phosphorsäure, Essigsäure, Natronlauge oder Triethylamin). Der jeweils günstigste pH-Wert kann durch einfache Reihenversuche leicht ermittelt werden.

Die in dieser ersten Phase gewonnenen Zellaggregationen werden in der zweiten Phase bei der Herstellung der Zellgranulate eingesetzt.

Die zweite Phase des Verfahrens, die Bildung der Zellgranulate, ist gekennzeichnet durch den intensiven Zuwachs an Biomasse eines Zellaggregates im Verlauf einer Fermentation. Zur Gewinnung von stabilen Zellgranulaten wird diese Fermentation zweckmäßigerweise in der Weise durchgeführt, daß es zur Ausbildung eines intensiv verzweigten Zellwachstums kommt, wodurch die Bildung eines gewebeartigen Zellverbandes gefördert wird.

Die Kultivierung der Mikroorganismen erfolgt hierbei unter aeroben Bedingungen und kann gemäß der allgemeinen üblichen Methoden wie unter Verwendung von Schüttelkulturen, z.B. als Schüttelkolben oder als Submerskultivierung in belüfteten Fermentern, z.B. in üblichen Submersfermentationstanks durchgeführt werden. Die Fermentation kann in diskontinuierlichen oder kontinuierlichen Verfahren erfolgen, vorzugsweise jedoch in diskontinuierlichem Betrieb.

Die Produktion der Zellgranulate kann in einem einstufigen oder zwei- bzw. mehrstufigen Verfahren, vorzugsweise jedoch in einem einstufigen Verfahren durchgeführt werden.

Die Gewinnung der Vorkultur (Inokulum) erfolgt nach den üblichen Methoden in Oberflächenkulturen, z.B. als Schrägröhrchen, auf Nähragarplatten oder auf als Nährsubstrat verwertbaren Trägermaterialien, bzw. in Flüssigkultur, wie z.B. im Schüttelkolben.

Das erfindungsgemäße Fermentationsverfahren wird in flüssigem Nährmedium, bevorzugt in wäßrigflüssigen Nährmedien durchgeführt. Hierbei eignen sich Nährmedien, die in ihrer Zusammensetzung die spezifischen Nährstoffansprüche des entsprechenden Mikroorganismus, wie z.B. Metarhizium anisopliae, abdecken. Das Nährmedium muß eine oder mehrere assimilierbare Kohlenstoff- und Stickstoffquellen sowie Mineralsalze enthalten, wobei diese Produkte in Form von definierten Einzelbestandteilen oder in Form von komplexen Gemischen wie sie insbesondere als biologische Produkte pflanzlichen oder tierischen Ursprungs verwendet werden. Als Kohlenstoffquellen kommen alle üblichen Kohlenstoffquellen in Frage. Beispeilsweise seien Kohlehydrate, insbesondere Polysaccharide, wie Stärke oder Dextrine, Disaccharide, wie Maltose oder Rohrzucker, Monosaccharide, wie Glucose oder Xylose, Zuckeralkohole, wie Mannit oder Glycerin sowie natürlich vorkommende Gemische wie Malzextrakt, Melasse oder Molke genannt. Als Stickstoffquellen kommen alle üblichen organischen und anorganischen Stickstoffquellen in Frage. Beispielsweise seien Aminosäuren, Eiweißstoffe, Eiweißhydrolysate, Nucleosidbasen sowie Sojabohnenmehl, Baumwollsamenmehl, Linsenmehl, Erbsenmehl, lösliche und unlöslichem pflanzliche Proteine, Maisquellwasser, Hefeextrakt, Peptone und Fleischextrakt sowie Ammoniumsalze und Nitrate, z.B. $NH_4Cl$, $(NH_4)_2SO_4$, $NaNO_3$ und $KNO_3$ aufgeführt. Die Mineralsalze, welche im Nährmedium enthalten sein sollten, liefern z.B. folgende Ionen:

$Mg^{++}$, $Na^+$, $K^+$, $Ca^{++}$, $NH_4^+$, $Cl^-$, $SO_4^{--}$, $PO_4^{---}$ und $NO_3^-$

sowie Ionen der üblichen Spurenelemente, wie Cu, Fe, Mn, Mo, Zn, Co, Ni. Falls die Kohlenstoff- oder Stickstoffquellen bzw. das verwendete Wasser nicht ausreichend diese Salze bzw. Spurenelemente enthalten, ist es zweckmäßig, das Nährmedium entsprechend zu ergänzen. Die Zusammensetzung der Nährmedien kann in weiten Bereichen variiert werden. Art und Zusammensetzung der Nährmedien werden im allgemeinen davon abhängig sein, welche Bestandteile jeweils besonders günstig zur Verfügung stehen. Im allgemeinen enthalten die Nährlösungen vorzugsweise etwa 0,5 bis 8 %, insbesondere 0,6 bis 6 % Kohlenstoffquellen, vorzugsweise etwa 0,5 bis 4 %, insbesondere 0,5 bis 2 % Stickstoffquellen und vorzugsweise etwa 0,001 bis 0,5 %, insbesondere 0,003 bis 0,3 % Mineralsalze.

Bei der Durchführung des Verfahrens kann es günstig sein, zu Beginn die Kultivierung nur relativ geringe Konzentrationen der löslichen Nährlösungsbestandteile zu verwenden und dann im Laufe der ersten Kultivierungsphase durch häufigere Zusätze dieser Bestandteile in Form steriler, relativ konzentrierter Lösungen dem Fermentationsansatz fraktioniert zuzufüttern.

Zur Steigerung der Stabilität der Granulate kann es sich als günstig erweisen, wenn dem Nährmedium komplexbildende Substanzen zugesetzt werden. Als komplexbildende Substanzen eignen sich anorganische und organische chelatbildende Verbindungen, wie z.B. Ethylendiaminotetraacetat, Diaminocyclohexan-N,N-tetraacetat, Diethylentriaminpentaacetat, Cyanide, Citrate mit und ohne komplexierten Metallionen. Die komplexbildenden Substanzen werden vorzugsweise in einer Konzentration von 0,5 bis 10 mM, besonders bevorzugt von 1,0 bis 5,0 mM eingesetzt.

Der pH-Wert der wachsenden Kultur sollte in einem Bereich gehalten werden, der die Initiation der Zellaggregation und die Bildung von Granulaten mit hinreichender Stabilität bei maximalen Zellwachstum gewährleistet. Der Fachmann kann durch die üblichen Methoden (Reihenversuche) auf einfache Weise die für die Zellaggregation und die Granulatproduktion optimalen pH-Wert-Bereich ermitteln. Bei der Durchführung des Fermentationsverfahrens kann es günstig sein, den pH-Wert in Abhängigkeit von der Fermentationsphase zu verändern, um eine maximale Biomasseproduktion zu fördern. Ein zu starker Abfall des pH-Wertes im den sauren Bereich kann durch Zusätze von Basen, z.B. NaOH oder $CaCO_3$ ausgeglichen werden. Wie in der Fermentationstechnologie üblich, kann auch eine automatische pH-Regulierung durchgeführt werden, bei der sterile organische oder anorganische Säuren oder Laugen in Abständen in die Kultur eingespritzt werden.

Die Sauerstoffversorgung der wachsenden Kultur ist zweckmäßigerweise derart sicherzustellen, daß der Sauerstoff nicht zum wachstumslimitierenden Faktor der Mikroorganismen wird. Die Sauerstoffversorgung der Kulturen wird üblicherweise durch Schütteln, z.B. im Schüttelkolben oder durch Belüftung in Verbindung mit Rührung der Fermentationstanks erfolgen.

Bei dem Erfindungsgemäßen Verfahren erfolgt die Kontrolle des Granulatdurchmessers bzw. der Granulatstabilität durch die Wahl der Schüttel- oder Rotationsgeschwindigkeit der Kulturkolben, wobei die Anzahl der Umdrehungen je nach Granulatdurchmesser bzw. Granulatstabilität vorzugsweise in einem Bereich von 50 UPM bis 250 UPM, besonders bevorzugt in einem Bereich von 100 bis 200 UPM gehalten wird. Bei der Kultivierung der Mikroorganismen in Fermentationstanks wird die Rührgeschwindigkeit vorzugsweise in einem Bereich von 30 bis 800, insbesondere von 50 bis 500 UPM gehalten (UPM bedeutet Umdrehungen pro Minute). Der Fachmann kann durch einfache Reihenversuche die jeweils günstigste Schüttel- oder Rührgeschwindigkeit leicht ermitteln, die zur Bildung von Zellgranulaten mit gewünschtem Durchmesser bzw. Stabilität führt.

Bei der Durchführung des Verfahrens kann es günstig sein, die Schüttel- oder Rührgeschwindigkeit zu Beginn der Kultivierung in einem sehr niedrigen Bereich zu halten und nach deutlich einsetzendem Zuwachs der Biomasse in einen Bereich höherer Schüttel- oder Rührgeschwindigkeit umzuschalten.

Die Temperatur zur Initiation der Zellaggregation bzw. zur Herstellung der Zellgranulate wird zweckmäßigerweise in einem Bereich gehalten, der ein maximales Zellwachstum erlaubt, vorzugsweise jedoch in einem Bereich von 10° bis 30°C.

Wie allgemein bei mikrobiologischen Verfahren sollten Fremdinfektionen der Kulturmedien vermieden werden. Hier werden die üblichen Vorkehrungen getroffen, wie Sterilisation der Nährmedien, der Kulturgefäße sowie der für die Belüftung notwendigen Luft. Zur Sterilisation der Vorrichtungen können z.B. die Dampf- als auch die Trockensterilisation verwendet werden, wobei die Temperaturen vorzugsweise bei 100° bis 140°C, insbesondere bis 120° bis 130°C liegen.

Falls bei der Kultivierung in unerwünschter Menge Schaum entsteht, können die üblichen chemischen Schaumdämpfungsmittel, z.B. flüssige Fette und Öle, Öl-Wasser-Emulsionen, Paraffine, höhere Alkohole, wie Octadecanol, Siliconöle, Polyoxyethylen- bzw. Polyoxypropylenverbindungen (z.B. in Mengen bis etwa 1 %) zugesetzt werden. Schaum kann auch mit Hilfe der üblichen mechanischen Vorrichtungen (welche z.B. Zentrifugalkräfte benutzen) gedämpft oder beseitigt werden.

Das Fermentationsende wird durch die Produktion der Biomasse determiniert. Die Kultivierung wird aus Gründen der Stabilität der Granulate bzw. der Erhaltung der Lebensfähigkeit der Zellen in den Granulaten günstigerweise vor oder zum Zeitpunkt der maximalen Biomasseproduktion abgebrochen. Die Festlegung des Zeitpunktes für das Produktionsende kann durch die üblichen Methoden der Biomassebestimmung durch den Fachmann leicht erfolgen. Durch in einfacher Weise ermittelbare fermentationsspezifische Kenndaten, wie pH-Wert, Sauerstoffpartialdruck, Kohlendioxidpartialdruck oder Konzentration an assimilierbaren Nährstoffbestandteilen kann das technische Produktionsverfahren gesteuert werden.

Die Abtrennung der Zellgranulate von dem Nährmedium kann in üblicher Weise erfolgen, z.B. durch Filtration über einem Sieb oder seibartigen Geweben mit entsprechender Porenweite, durch Filtration, Zentrifugation oder Separation. Zur Vermeidung von Kontaminationen der Zellgranulate durch unerwünschte Mikroorganismen, die durch ihre Stoffwechselaktivitäten eine Verminderung der Qualität bzw. eine Zerstörung des Produktes verursachen könnten, wird die Konzentrierung der Zellgranulate (und gegebenenfalls auch die weitere Verarbeitung) zweckmäßigerweise unter sterilen Bedingungen, wie z.B. in sterilisierten Separatoren durchgeführt.

Zur leichteren weiteren Verarbeitung der Zellgranulate kann es zweckmäßig sein, der zur konzentrierenden Biomasse üblicherweise verwendete Materialien zuzusetzen, die ein Verklumpen der Zellgranulate während des Konzentrierungsverfahrens verhindern. Hierzu eignen sich oberflächenneutralisierende Materialien, wie z.B. die tonartigen Materialien Bentonite, Talkum, Pyrophylite, Celit, Kalk, Kaolin, Attapulgit oder andere synthetische Silikate.

Die Trocknung der Zellgranulate erfolgt durch Dehydratation der Mikroorganismen. Hierbei können die üblichen Methoden zur Trocknung von Biomasse mittels Wärmeübertragung durch Konvektion, wie z.B. die Verfahren der Zerstäubungs-, Strom- und Fließbetttrocknung oder mittels Wärmeübertragung durch Kontakt, wie z.B. die Verfahren der Teller-, Schaufel-, Taumel-, Band-, Walzen-, Vakuumkammer- und Vakuumgefriertrocknung eingesetzt werden. Der Trocknungsprozeß kann auch aus einer Kombination von zwei oder mehreren dieser Verfahren bestehen. Die Dehydratation der Zellgranulate wird im diskontinuierlichen oder kontinuierlichen Verfahren, vorzugsweise jedoch im diskontinuierlichen Verfahren durchgeführt. Die Verfahren sind im einzelnen derart auszugestalten, daß die Lebensfähigkeit der Zellen in den Granulaten über einen möglichst langen Zeitraum hinweg gewährleistet ist. Ferner ist bei der Trocknung der Zellgranulate zu beachten, daß die mechanische Belastung der Zellgranulate möglichst gering gehalten wird.

Das getrocknete Produkt sollte einen Wassergehalt von 0 bis 30 % (w/w), vorzugsweise jedoch von 2,5 bis 15 % (w/w) aufweisen. Der Wassergehalt (bezogen auf ein bei 100°C 12 Stunden getrocknetes Produkt) wird nach den üblichen Methoden bestimmt.

Zum Schutz der Zellen in den Granulaten vor Schädigungen, wie sie während des Trocknungsprozesses in Folge einer Temperaturerniedrigung oder Temperaturerhöhung bzw. durch eine zu intensive Dehydration entstehen können, kann es zweckmäßig sein, die Granulate vor der Trocknung mit entsprechenden Schutzsubstanzen vorzubehandeln. Hierzu eignen sich für diesen Verwendungszweck bekannte organische oder anorganische Substanzen, die in definierter Form oder als komplexes Gemisch eine protektive Wirkung erzielen, z.B. Polyalkohole, wie Zucker oder Glycerin. Die Behandlung der Zellen erfolgt mit den üblichen Methoden durch Tauchen, Waschen, Besprühen oder Mischen der Zellgranulate mit den Schutzmitteln.

Ein Schutz der Zellen in den Granulaten vor unkontrollierten Oxidationsreaktionen kann durch eine Behandlung der noch nicht getrockneten Zellgranulate mit untoxischen antioxidativen Stoffen, wie z.B.

Ascorbinsäure, 2,3-tert.-Butyl-4-hydroxy-anisol, 2,6-Di-tert.-Butyl-p-cresol, Propylgallate oder Nordihydroguaiaretsäure erreicht werden. Die Behandlung erfolgt mit Hilfe der üblichen Methoden durch Tauchen, Waschen, Besprühen oder Mischen der Granulate mit den Schutzmitteln.

Zur Entwicklung der biologischen Aktivität in der Schädlingsbekämpfung können die Zellgranulate vor der Trocknung auch mit Materialien behandelt werden, die die Rehydration der Zellen unterstützen. Hierzu eignen sich alle untoxische hygroskopisch wirkenden Materialien, insbesondere Polyalkohole wie Glycerin, Zucker, Zuckerpolymere oder Derivate der Zuckerpolymere.

Zur Aktivierung und Intensivierung der biologischen Wirkung insbesondere in der Schädlingsbekämpfung kann es zweckmäßig sein, daß die Zellgranulate vor der Trocknung mit Nährstoffen behandelt werden, die eine rasche Vermehrung der Mikroorganismen und somit eine dichtere Besiedlung des Wirkortes unterstützen. Als nährstoffartige Substanzen eignen sich alle assimilierbaren Kohlen- und Stickstoffquellen, wie sie auch zur Anzucht bzw. bei der Fermentation des entsprechenden Mikroorganismus genutzt werden können.

Zur Erhaltung einer langen Lebensfähigkeit der Mikroorganismen und zur Erreichung einer besonders guten Lagerfähigkeit der Zellgranulate kann es zweckmäßig sein, auf der Oberfläche der Zellgranulate, insbesondere von Pilzen mit fädigem Zellwachstum, wie z.B. Metarhizium anisopliae, die Bildung von Dauerstadien, wie z.B. Konidien, vor der Trocknung der Zellgranulate zu induzieren. Die Bildung der Dauerstadien erfolgt durch zusätzliche Inkubation der fermentativ gewonnenen Zellgranulate unter Bedingungen der Oberflächenkulturen, z.B. auf flachen Schalen, Wannen oder Blechen. Die Luftfeuchtigkeit wird hierbei durch die üblichen Methoden in einem Bereich von 100 % bis 40 % relative Luftfeuchte, vorzugsweise jedoch in einem Bereich von 100 % bis 80 % relative Luftfeuchte gehalten. Die Inkubation sollte bei Temperaturen nicht unter 10°C bzw. nicht über 30°C, vorzugsweise jedoch in einem Bereich von 20° bis 27°C durchgeführt werden. Die Bildung der Dauerstadien läßt sich in einfachster Weise durch die Pigmentierung der Zellgranulate verfolgen bzw. durch die üblichen Methoden der Mikroskopie.

Für eine gleichmäßige Bildung der Dauerstadien auf der Oberfläche der Zellgranulate kann es sich als günstig erweisen, wenn die Zellgranulate in bestimmten zeitlichen Abständen mechanisch bewegt werden, wie z.B. durch Schütteln.

Zur Intensivierung der Dauerstadienbildung kann es sich als zweckmäßig erweisen, die Zellgranulate vor der Inkubation in Oberflächenkulturen mit kohlenstoff- oder insbesondere stickstoffhaltigen Nährstoffen, wie Zucker, Aminosäure, z.B. Tryptophan, Glutamat, Histidin, Aspartat oder proteinhaltigen Materialien zu behandeln. Die Nährstoffe werden in definierter Form oder als komplexe Gemische eingesetzt, wobei die optimalen Konzentrationen nach den üblichen Methoden ermittelt werden. Die Behandlung erfolgt mit den üblichen Methoden durch Tauchen, Waschen oder Besprühen der noch nicht getrockneten Zellgranulate.

Die Inkubation der Zellgranulate zur Bildung von Dauerstadien wird zur Vermeidung von Kontaminationen unerwünschter Mikroorganismen zweckmäßigerweise unter sterilen Bedingungen, wie z.B. in sterilisierten Behältern erfolgen.

Die Konservierung solcherart mit Dauerstadien behafteten Zellgranulate erfolgt, wie oben angegeben, durch Dehydration der Mikroorganismen. Hierbei ist es zweckmäßig darauf zu achten, daß zur Vermeidung einer Abtrennung der Dauerstadien die mechanische Beanspruchung der Zellgranulate möglichst gering gehalten wird. Unter diesen Voraussetzungen beiten sich für die Trocknung in erster Linie die Verfahren der Wärmeübertragung durch Kontaktverfahren, wie z.B. die Verfahren der Band-, Walzen-, Vakuumkammer- oder Vakuumgefriertrocknung an.

Die Lagerung der erfindungsgemäßen Zellgranulate erfolgt in geschlossenen Behältern unter trockenen Bedingungen, vorzugsweise bei Temperaturen zwischen 0° und 25°C. Zur Erhaltung der Lebensfähigkeit der Zellen in den Granulaten kann es zweckmäßig sein, die Granulate unter Ausschluß von Sauerstoff zu lagern, wie z.B. durch Lagerung unter einer Atmosphäre aus Stickstoff, Kohlendioxid oder anderen inerten Gasen, bzw. aus Gasgemischen der genannten Gase, ferner kann der Ausschluß des Sauerstoffs durch Verpackung der Zellgranulate unter Bedingungen des Unterdrucks erreicht werden.

Die erfindungsgemäßen Schädlingsbekämpfungsmittel können bei der Verwendung entsprechender Mikroorganismen zur Bekämpfung von tierischen Schädlingen, vorzugsweise Arthropoden und Nematoden, insbesondere Insekten und Spinnentieren angewendet werden, die in der Landwirtschaft, in Forsten, im Gartenbau, im Vorrats- und Materialschutz sowie auf dem Hygienesektor vorkommen.

Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

EP 0 268 177 B1

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der heteroptera z.B. Eurygaster spp., Dysdercus intermedius, Piesma quadrata, Cimex lectularius, Rhodnius prolixus, Triatoma spp.

Aus der Ordnung der homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis captitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Bevorzugt werden die neuen Schädlingsbekämpfungsmittel zur Bekämpfung von Insekten und Nematoden, vorzugsweise Insekten, welche im oder am Boden (bzw. in Bodennähe) vorkommen (Bodeninsekten) verwendet.

Die Schädlingsbekämpfungsmittel können auch in Fallen, gegebenenfalls nach der Zumischung von Köderstoffen oder Lackstoffen eingesetzt werden.

Die erfindungsgemäßen Schädlingsbekämpfungsmittel können bei der Verwendung entsprechender Mikroorganismen zur Bekämpfung von schädlichen Mikroben (Pilze und Bakterien) eingesetzt werden.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Pseudomonas-Arten, wie beispielsweise Pseudomonas solanacearum;

Pythium-Arten, wie beispielsweise Pythium ultimum;

Phytophthora-Arten, wie beispielsweise Phytophthora cactorum;

Fusarium-Arten, wie beispielsweise Fusarium oxysporum;

Botrytis-Arten, wie beispielsweise Botrytis cinerea;

Pseudocercosporella-Arten, wie beispielsweise Pseudocercosporella herpotrichoides;

Rhizoctonia-Arten, wie beispielsweise Rhizoctonia solani;

Sclerotium-Arten, wie beispielsweise Sclerotium rolfsii;
Sclerotinia-Arten, wie beispielsweise Sclerotinia sclerotiorum;
Verticillium-Arten, wie beispielsweise Verticillium alboatrum;
Phialophora-Arten, wie beispielsweise Phialophora cinerescens;
Phomopsis-Arten, wie beispielsweise Phomopsis sclerotioides.

Die erfindungsgemäßen Schädlingsbekämpfungsmittel (bzw. Pflanzenbehandlungsmittel) können beim Einsatz geeigneter Mikroorganismen auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Die Selektivität der Herbizide hängt im wesentlichen von der jeweils angewendeten Menge ab.

Die erfindungsgemäßen Mittel können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Mittel ist jedoch keineswegs auf diese Gattungen beschränkt, sondern erstreckt sich in gleicher Weise auch auf andere Pflanzen.

Die Mittel eignen sich in Abhängigkeit von der Konzentration zur Unkrautbekämpfung z.B. auf Industrie- und Gleisanlagen und auf Wegen und Plätzen mit und ohne Baumbewuchs, ebenso zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen.

Die erfindungsgemäßen Mittel können als solche oder in ihren Formulierungen (vorzugsweise als solche) auch in Mischung mit bekannten anderen Schädlingsbekämpfungsmitteln wie Insektiziden, Akariziden, Nematiziden, Schutzstoffen gegen Vogelfraß, Pflanzennährstoffen, Herbiziden und Bodenstrukturverbesserungsmitteln Verwendung finden.

Die Anwendung der erfindungsgemäßen Mittel geschieht in üblicher Weise, vorzugsweise durch Streuen. Besonders bevorzugt werden die erfindungsgemäßen Mittel ohne eine weitere Formulierung angewendet, wobei die Anwendung vorzugsweise durch Streuen erfolgt.

Die angewandte Menge der Mittel kann in einem größeren Bereich schwanken. Sie hängt im wesentlichen von der Art des gewünschten Effektes und der Art der eingesetzten Mikroorganismen ab. Im allgemeinen liegen in den Bereichen Landwirtschaft, Forsten und Gartenbau die Aufwandmengen zwischen 0,1 und 50 kg Mittel pro Hektar Bodenfläche, vorzugsweise zwischen 1 und 25 kg pro ha.

Vorzugsweise dienen die erfindungsgemäßen Schädlingsbekämpfungsmittel zur Bodenbehandlung. Hierbei werden vorzugsweise Schädlinge bekämpft, welche im Bereich des Bodens vorkommen können.

Die vorliegende Erfindung soll anhand der folgenden Beispiele erläutert werden.

Anmerkung die durch eine "CBS-Nr." gekennzeichneten Stämme wurden aus der Sammlung des Centraalbureau voor Schimmelcultures (CBS), Oosterstraat 1, NL-3740 AG Baarn, Niederlande, erhalten.

A) Herstellung der Zellgranulate vom Beispiel von Metarhizium anisopliae P 0001 (DSM 3884)

1. 10.0 Liter-Fermentation zur Herstellung der Zellgranulate

Metarhizium anisopliae P 0001 (DSM 3884) wird als Stammkultur auf Schrägröhrchen mit Malzextrakt-Glucose-Pepton-Nähragar (Malzextrakt 20,0 g; Glucose 20,0 g; Pepton 1,0 g; Agar 15,0 g ad 1000 ml Wasser, pH 7,5) gehalten. Die Schrägröhrchen werden im Kühlschrank bei 4°C gelagert.

Die Gewinnung von Konidien des Metarhizium anisopliae Stammes als Inokulum für die Fermenterkulturen erfolgt auf Malzextrakt-Glucose-Pepton-Agarplatten, die mit einer Konidiensuspension von Schrägröhrchen angeimpft für 15 bis 16 Tage bei einer Temperatur von 25°C inkubiert werden.

Die Vorkulturen für die Fermentationen werden in 1,0 Liter Erlenmeyerkolben angezogen, die mit 100 ml Nährlösung gefüllt sind. Die Nährlösung setzt sich wie folgt zusammen:

| Glucose | 10,0 | g |
|---|---|---|
| Hefeautolysat (Ohly) | 10,0 | g |
| KH$_2$PO$_4$ | 1,74 | g |
| FE-III-Citrat | 0,28 | g |
| MnSO$_4$ × H$_2$O | 0,031 | g |
| ZnSO$_4$ × 7 H$_2$O | 0,009 | g |
| CuSO$_4$ × 5 H$_2$O | 0,0057 | g |
| MgCl$_2$ × 6 H$_2$O | 0,406 | g |
| ad 1000 Milliliter H$_2$O, pH 7,5. | | |

Zur Vermeidung von Schaumbildung wird dem Nährmedium ein Silikonöl (Baysilone E, Warenzeichen der Bayer AG, Leverkusen, Bundesrepublik Deutschland) (30 %, v/v; 0,5 ml/l Nährlösung) zugesetzt. Die Nährlösung wird mit einer Konidiensuspension angeimpft, die durch Abschwemmen von Malzextrakt-Glucose-Pepton-Agarplatten mit einer wäßrigen Lösung (1,0 %, v/v) eines nichtionischen oberflächenaktiven Mittels (Polyoxyethylenderivat von Sorbitanhydrid, Tween 20) (Warenzeichen der ICI America Inc. USA) gewonnen wurde. Der Konidientiter in den Vorkulturen beträgt 10$^6$ Konidien pro Milliliter Nährlösung. Nach dem Animpfen werden die Kulturen für 24 Stunden auf einem Rundschüttler bei 100 Umdrehungen pro Minute auf einer Temperatur von 25°C inkubiert.

Die Herstellung der Zellgranulate wird in einem 15,0 Liter Fermenter durchgeführt, der 10,0 Liter der obengenannten Nährlösung enthält. Das Nährmedium wurde 45 Minuten bei 121°C sterilisiert.

Die Fermenter werden mit 3,0 %, v/v, Vorkultur angeimpft. Während der Fermentation werden die folgenden Anzuchtbedingungen eingehalten:

Temperatur 25°C

Rührgeschwindigkeit 400 Umdrehungen pro Minute

Belüftungsrate 5 Liter Luft pro Minute

Die Fermentationen sind nach 60 bis 80 Stunden abgeschlossen.

2. Trocknung der Zellgranulate

Die bei der oben beschriebenen Fermentation erhaltenen Zellgranulate von Metarhizium anisopliae werden durch Absieben über einem Kunststoffgewebe mit einem Porendurchmesser von 0,1 mm von der Fermentationsbrühe abgetrennt. Durch Absaugen der Zellgranulate über eine Filtriernutsche, die mit einer Membranpumpe verbunden ist, wird ein zusätzlicher Anteil an ungebundener Fermentationsflüssigkeit abgetrennt.

Die Trocknung der Zellgranulate wird in einem Wirbelschichtgranulator mit einem Füllvolumen von 16,5 Litern durchgeführt. Die Zellgranulate werden in Portionen zu je 200 g in den Wirbelschichtgranulator gefüllt und im Luftstrom bei einer Strömungsrate von 1300 Litern pro Minute getrocknet. Die Temperatur der zugeführten Luft beträgt 40°C. Der Trocknungsprozeß wird durch die regelmäßige Bestimmung des Wassergehaltes der Zellgranulate verfolgt. Bei einem Wassergehalt der Zellgranulate von 10 % (w/w, bezogen auf ein bei 100°C 12 Stunden getrocknetes Produkt) wird der Trocknungsprozeß beendet.

Die Zellgranulate werden unter trockenen Bedingungen bei Raumtemperatur gelagert.

B) Herstellung von Zellgranulaten mit einer Konidienbildung an der Granulatoberfläche am Beispiel von Metarhizium anisopliae P 0001 (DSM 3884)

Die Herstellung und Aufarbeitung von Zellgranulaten erfolgt wie unter Beispiel A) beschrieben. Nach der Abtrennung der Fermentationsbrühe werden die Zellgranulate mit einer konzentrierten Glucoselösung (10 %, w/v; 100 ml Glucoselösung für 50 g feuchte Zellgranulate) gewaschen. Anschließend wird die nicht gebundene Glucoselösung über eine Filtriernutsche abgesaugt.

Die so behandelten Zellgranulate werden für 60 bis 70 Stunden in einer Feuchtkammer, relative Luftfeuchtigkeit 100 %, bei einer Temperatur von 25°C inkubiert. Die Bildung der Konidien auf der Granulatoberfläche läßt sich über die Intensität der grünen Pigmentierung verfolgen.

C) 10,0 Liter Fermentation zur Herstellung von Zellgranulaten am Beispiel von Gliocladium roseum (CBS 595.75)

Die Stammhaltung und die Gewinnung jvon Konidien als Inokulum für die Vorkulturen erfolgt bei Gliocladium roseum (CBS 595.75) nach dem im Beispiel 1 für Metarhizium anisopliae P 0001 beschriebenen Verfahren.

Die Vorkulturen für die Fermenterkulturen werden in 1,0 Liter Erlenmeyerkolben angezogen, die mit 100 Milliliter Nährlösung gefüllt sind. Die Nährlösung setzt sich wie folgt zusammen:

| | | |
|---|---|---|
| Glucose | 10,0 | g |
| Hefeextrakt | 10,0 | g |
| ad 1000 Milliliter $H_2O$, pH 6,0 | | |

Die Vorkulturen werden mit einer Konidiensuspension angeimpft, die durch Abschwemmen von Agarplattenkulturen (Malzextrakt-Glucose-Pepton-Agar) gewonnen wurden. Der Konidientiter in den Vorkulturen beträgt $10^6$ Konidien pro Milliliter Nährlösung. Nach dem Animpfen werden die Kulturen für 24 Stunden auf einem Rundschüttler bei 150 Umdrehungen pro Minute und einer Temperatur von 25°C inkubiert.

Die Herstellung der Zellgranulate wird in einem 15,0 1 Fermenter durchgeführt, der mit 10,0 1 Nährlösung mit folgender Zusammensetzung gefüllt ist:

| | | |
|---|---|---|
| Stärke | 10,0 | g |
| Caseinhydrolysat | 10,0 | g |
| ad 1000 Milliliter $H_2O$, pH 6,0 | | |

Das Nährmedium wird 45 Minuten bei 121°C sterilisiert. Die Fermenter werden mit 1,5 %, v/v Vorkultur angeimpft. Während der Fermentation werden folgende Parameter eingehalten:

| | |
|---|---|
| Temperatur: | 25°C |
| Belüftungswerte: | 5,0 l Luft pro Minute |
| Rührgeschwindigkeit: | 100 UPM für 24 Stunden nach Inokulation |
| | 200 UPM 24. Stunde bis Fermentationsende |

Die Fermentationen sind nach 60 bis 80 Stunden abgeschlossen.

D) Gewinnung von Zellgranulaten verschiedener Mikroorganismen in Schüttelkulturen

Die Bildung von Zellgranulaten kann durch Einstellung von bestimmten Rahmenbedingungen, die in Reihenversuchen ermittelt wurden, in Schüttelkulturen erfolgen. Als Beispiele wurden Mikroorganismen, insbesondere die Vertreter der Deuteromyceten gewählt, von denen eine biologische Aktivität bei der Schädlingsbekämpfung bekannt ist.

Die Stammhaltung und die Gewinnung der Konidien für das Inokulum der Schüttelkulturen erfolgt nach dem für Metarhizium anisopliae P 0001 (Beispiel 1) beschriebenen Verfahren.

Die Bildung der Zellgranulate erfolgt in Erlenmeyerkolben mit einem Gesamtvolumen von 1,0 1, die jeweils mit 100 Milliliter der angegebenen Nährlösung gefüllt sind. Der Kulturansatz wurde 20 Minuten bei 121°C autoklaviert.

Die Kulturansätze werden mit einer Konidiensuspension angeimpft, die durch Abschwemmen von Agarplattenkulturen gewonnen wird. Das Konidientiter beträgt nach dem Animpfen $10^6$ Konidien pro Milliliter Nährlösung. Nach dem Animpfen erfolgt die Inkubation auf einem Rundschüttler mit einer Amplitude von 5,0 cm; die Schüttelgeschwindigkeit (Umdrehungen pro Minute) ist in der Tabelle angegeben. Die Temperatur wird bei 25°C konstant gehalten.

In Tabelle 1 sind beispielhaft die Bedingungen zusammengefaßt, unter denen die verschiedenen Mikroorganismen zur Bildung von Zellgranulaten gebracht werden.

Tabelle 1) Rahmenbedingungen für die Bildung von Zellgranulaten bei diversen Mikroorganismen

Tabelle 1) Rahmenbedingungen für die Bildung von Zellgranulaten verschiedener Mikroorganismen

| Mikroorganismus | Indikation | Nährmedium | pH-Wert | Schüttel-geschwindigkeit |
|---|---|---|---|---|
| Gliocladium viride (CBS 137.79) | Fungizid | Standard 1* | 7,5 | 100 UPM |
| Aphanocladium album (CBS 376.77) | Fungizid | Standard 1** | 6,0 | 50 UPM |
| Gliocladium solani (CBS 227.80) | Fungizid | Standard 1 | 7,5 | 50 UPM |
| Gliocladium virens (CBS 344.47) | Fungizid | Standard 2 | 7,5 | 100 UPM |
| Coniothyrium minitans (CBS 641.80) | Fungizid | Standard 2 | 7,5 | 100 UPM |
| Meria coniospora (= Drechmeria coniospora) (CBS 615.82) | Nematizid | Standard 2 | 7,5 | 100 UPM |
| Verticillium lecanii (CBS 318.70 C) | Insektizid | Dextrin, 1,0 % w/v Casein, 1,0 % w/v | 4,0 | 75 UPM |
| Verticillum bulbillosum (CBS 571.78) | Fungizid | Standard 1 | 7,5 | 100 UPM |
| Penicillium expansum (CBS 481.84) | Fungizid | Standard 1 | 7,5 | 100 UPM |
| Penicillum oxalium (CBS 460.67) | Fungizid | Standard 2 | 7,5 | 100 UPM |
| Colletotrichum gloeosporioides (CBS 796.72) | Herbizid | 1 % Casein in 1 % Glucose Wasser | 7,0 | 100 UPM |

Anmerkung: *) Standardmedium 1: Kartoffelinfusion von 200 g Kartoffeln;
Glucose 20,0 g, ad 1000 ml Wasser

**) Standardmedium 2: Zusammensetzung siehe Beispiel 1 für Metarhizium
anisopliae P 0001

EP 0 268 177 B1

Die biologische Wirksamkeit der erfindungsgemäßen Zellgranulate soll anhand der folgenden Beispiele erläutert werden:

Beispiel 1

Testinsekt:Agrotis segetum, Larven 3, Stadium

Testzellgranulat:Zellgranulat aus Metarhizium anisopliae gemäß Beispiel A mit einer Korngröße von 0,5 bis 1,0 mm (Durchmesser)

Das Zellgranulat wird innig mit Feldboden (Wassergehalt: 15 Vol.-%) vermischt. Die Konzentration des Zellgranulats im Boden wird dabei in Granulatgewicht pro Volumeneinheit Boden (ppm = mg/l) angegeben.

Man füllt den mit Zellgranulat behandelten Boden in paraffinierte Papptöpfe, gibt unverzüglich die Testtiere in den Boden, verschließt die Versuchstöpfe und stellt sie während der Versuchsdauer bei einer Temperatur von 20°C auf. Während der Versuchsdurchführung werden als Futterquelle für die Testinsekten Möhrenscheiben ad libitum angeboten. Nach 10 bis 20 Tagen wird der Wirkungsgrad des Zellgranulats durch Auszählung der toten und lebenden Testinsekten in % (Abbott) bestimmt. Der Wirkungsgrad ist 100 %, wenn alle Testinsekten getötet worden sind, er ist 0 %, wenn noch genau so viele Testinsekten leben, wie in der Kontrolle.

| Ergebnis: | |
| --- | --- |
| Konzentration der Zellgranulats in ppm | Wirkungsgrad in % |
| 10 000 | 100 |

Beispiel 2

Testinsekt:Diabrotica balteata, Larven 2. Stadium

Testzellgranulat:Zellgranulat aus Metarhizium anisopliae P 0001 mit einer Korngröße von 1,0 mm (Durchmesser)

Das Zellgranulat wird innig mit Feldboden (Wassergehalt: 15 Vol.-%) vermischt. Die Konzentration des Zellgranulats im Boden wird dabei in Granulatgewicht pro Volumeneinheit Boden (ppm = mg/l) angegeben.

Man füllt den mit Zellgranulat behandelten Boden in paraffinierte Papptöpfe, gibt unverzüglich die Testtiere in den Boden. Als Futterquelle für die Testinsekten werden vorgekeimte Maiskörner mit in den behandelten Boden ausgesät. Man verschließt die Versuchstöpfe und stellt sie während der Versuchsdauer bei einer Temperatur von 20°C auf. Nach 10 bis 20 Tagen wird der Wirkungsgrad des Zellgranulats durch Auszählung der toten und lebenden Testinsekten in % (Abbott) bestimmt. Der Wirkungsgrad ist 100 %, wenn alle Testinsekten getötet worden sind, er ist 0 %, wenn noch genau so viele Testinsekten leben, wie in der Kontrolle.

| Ergebnis: | |
| --- | --- |
| Konzentration der Zellgranulate in ppm | Wirkungsgrad in % |
| 10 000 | 100 |

Beispiel 3

Testinsekt:Tenebrio molitor, Larven 3, Stadium

Testzellgranulat:Zellgranulat aus Metarhizium anisopliae gemäß Beispiel A mit einer Korngröße von 0,5 bis 1,0 mm (Durchmesser)

Das Zellgranulat wird innig mit Feldboden (Wassergehalt: 12 Vol.-%) vermischt. Die Konzentration des Zellgranulats im Boden wird dabei in Granulatgewicht pro Volumeneinheit Boden (ppm = mg/l) angegeben.

Man füllt den mit Zellgranulat behandelten Boden in paraffinierte Papptöpfe, gibt unverzüglich die Testtiere in den Boden, verschließt die Versuchstöpfe und stellt sie während der Versuchsdauer bei einer Temperatur von 20°C auf. Nach 10 Tagen wird der Wirkungsgrad des Zellgranulats durch Auszählung der toten und lebenden Testinsekten in % (Abbott) bestimmt. Der Wirkungsgrad ist 100 %, wenn alle Testinsekten getötet worden sind, er ist 0 %, wenn noch genau so viele Testinsekten leben, wie in der Kontrolle.

| Ergebnis: | |
|---|---|
| Konzentration der Zellgranulate in ppm | Wirkungsgrad in % |
| 5000 | 100 |

### Beispiel 4

Testpathogen:Fusarium colmorum
Testpflanze:Triticum aestivum cv. Vulka
Testzellgranulat:Zellgranulat aus Aphanocladium album CBS 276.77 bzw.
Verticillium bulbillosum CBS 571.78 gemäß Beispiel D mit einer Korngröße von 0,5-1,0 mm Gewächshaus-Einheitserde (Fa. Balster, D-5758 Fröndenberg) wird in Plastikschalen gefüllt und durch Sprühen einer Sporensuspension von Fusarium culmorum mit dem Pathogen verseucht. Auf diese Erde werden die Weizensamen ausgesät. Mit der Aussaat werden die Zellgranulate breitwürfig auf der Erde verteilt und die Schalen anschließend mit Erde angedeckt und angegossen. Während der Versuchsdurchführung werden die Schalen bei 20° bis 22°C im Gewächshaus gehalten und nach Bedarf bewässert.

Nach 2 bis 4 Wochen wird der Wirkungsgrad in % (Abbott) durch Auszählen der gesunden und der erkrankten Pflanzen bestimmt. Der Wirkungsgrad ist 100, wenn alle Pflanzen gesund sind, es ist 0, wenn die gleiche Anzahl Pflanzen wie in der Kontrolle erkrankt ist.

| Ergebnis | | |
|---|---|---|
| Aufwandmenge des Zellgranulates in g/m$^2$ | Wirkungsgrad (% Abbott) | |
| | A. Album | V. bulbillosum |
| 30 | 83 | 73 |

### Beispiel 5

Testpathogen:Rhizoctonia solani
Testpflanze:Pisum sativum cv. Wunder von Kelvedon
Testzellgranulat:Zellgranulat aus Aphanocladium album CBS 376.77 gemäß Beispiel D mit einer Korngröße von 0,5-1.0 mm

Auf Gewächshaus-Einheitserde (Fa. Balster, D-5758 Fröndenberg), die in Plastikschalen gefüllt wird, werden Erbsensamen ausgesät. Mit der Aussaat werden die Zellgranulate breitwürfig auf der Erde verteilt. Die Schalen werden dann mit Erde, die mit dem Pathogen verseucht ist, abgedeckt. Nach dem Angießen wird der Versuch bei 20 bis 22°C im Gewächshaus gehalten und nach Bedarf bewässert.

Nach 2 bis 4 Wochen wird der Wirkungsgrad in % (Abbott) durch Auszählen der gesunden und der erkrankten Pflanzen bestimmt. Der Wirkungsgrad ist 100, wenn alle Pflanzen gesund sind; est ist 0, wenn die Pflanzen in gleichem Maße wie in der Kontrolle erkrankt sind.

| Ergebnis | |
|---|---|
| Aufwandmenge des Zellgranulates in g/m$^2$ | Wirkungsgrad (% Abbott) |
| 30 | 59 |

### Beispiel 6

Testpathogen:Fusarium oxysporum f. sp. lycopersici
Testpflanze:Lycopersicon esculentum cv. Fremdgens Rheinlands Ruhm
Testzellgranulat:Zellgranulat aus Gliocladium roseum CBS 595.75 gemäß Beispiel C) mit einer Korngröße von 0,5 bis 1,0 mm

Man füllt Gewächshaus-Einheitserde (Fa. Balster, D-5758 Fröndenberg) in Plastiktöpfe und formt mit einem Pikierstab ein Pflanzloch. Vor der Pflanzung werden Konidien des Pathogens in wäßriger Suspension in die Erde gegeben. In die Pflanzlöcher werden die Zellgranulate von G. roseum verteilt, danach werden unverzüglich 3 bis 4 Wochen alte Tomatensämlinge eingepflanzt und angegossen. Während der Versuchsdurchführung werden die Pflanzen bei 20° bis 22°C im Gewächshaus gehalten und nach Bedarf mit Wasser versorgt.

Nach 3 bis 4 Wochen erfolgt die Auswertung des Versuches durch

a) Ermitteln des Pflanzenwachstums im Verhältnis zur befallsfreien Kontrolle

b) Bonitieren des Pflanzenhabitus mit einem Schema von 0 bis 5 (0 = keine Symptome, 5 = Pflanze abgestorben)

c) Ermitteln des verbräunten Stengelquerschnittes in Prozent. Der letzte Wert wird zur Ermittlung des Wirkungsgrades in % (Abbott) herangezogen. Der Wirkungsgrad ist 100, wenn keine Verbräunungen festzustellen sind; er ist 0, wenn das Maß der Verbräunungen dem der nicht behandelten Kontrolle entspricht.

| Ergebnis | | | |
| --- | --- | --- | --- |
| Konzentration des Zellgranulates in ppm (mg/l Erde) | % Pflanzenwuchs zur befallsfreien Kontrolle | Bonitur (0–5) | Wirkungsgrad (% Abbott) |
| 1.500 | 112 | 0 | 100 |

Beschreibung der Metarhizium anisopliae Stämme P 0001 (DMS 3884) und P 0003 (DSM 3885)

Die Metarhizium anisopliae Stämme P 0001 und P 0003 wachsen in Form von septierten, verzweigten Hyphensträngen. Bei Wachstum auf Agaroberflächen bilden die Pilze ein weißes, flaumiges Luftmycel aus.

Nach Ausbildung des Luftmycels setzt die Bildung der Dauerstadien, sogenannter Konidien ein, deren Länge 9,0 bis 12,0 μm, bzw. deren Durchmesser 2,0 bis 3,0 μm beträgt. Die Konidien sind in gleichförmigen Ketten angeordnet, wobei in der Regel mehrere Kettenstränge nebeneinander liegen. Die Pigmentierung der Konidien gibt den Kolonien von Metarhizium anisopliae Stamm P 0001 (DSM 3884) bei Wachstum auf Hafermehlagar eine braun-grüne, den von Metarhizium anisopliae Stamm P 0003 (DSM 3885) eine gelb-braune Färbung.

Bei Wachstum in Flüssigkulturen bilden die Pilze neben der fädigen Zellform (Hyphen) auch hefeartige Einzelzellstadien, sogenannte Blastosporen aus. Die Länge der Blastosporen beträgt 22,0 bis 25,0 μm, der Durchmesser 6,0 bis 8,0 μm.

## Patentansprüche

1. Schädlingsbekämpfungsmittel und Pflanzenbehandlungsmittel, dadurch gekennzeichnet, daß sie aus trägerfreien Zellgranulaten von Mikroorganismen die zur Schädlingsbekämpfung oder Pflanzenbehandlung geeignet sind, bestehen oder wenigstens ein trägerfreies Zellgranulat von solchen Mikroorganismen enthalten, wobei die Zellgranulate aus Pilzen oder Bakterien gebildet werden, die zur Myzelbildung fähig sind und wobei die Zellgranulate eine im wesentlichen kugelige Gestalt haben und Durchmesser von 0,05 bis 2 mm aufweisen.

2. Mittel gemäß Anspruch 1, wobei die Zellgranulate Nährstoffe, protektiv wirkende Substanzen, antioxidativ wirkende Stoffe und/oder die Rehydratation unterstützende Stoffe enthalten.

3. Mittel gemäß den Ansprüchen 1 und 2, wobei die Zellgranulate im wesentlichen an der Oberfläche eine erhöhte Menge an Mikroorganismen Dauerstadien aufweisen.

4. Mittel gemäß den Ansprüchen 1 bis 3, wobei die Zellgranulate aus Pilzen der Klasse der Deuteromycetes gebildet werden.

5. Schädlingsbekämpfungsmittel gemäß den Ansprüchen 1 bis 4, welche zur Bekämpfung von Arthropoden und Nematoden, vorzugsweise von Insekten geeignet sind, wobei die Zellgranulate durch Pilze der Gattung Metarhizium, vorzugsweise der Art Metarhizium anisopliae gebildet werden.

6. Schädlingsbekämpfungsmittel gemäß den Ansprüchen 1 bis 5, wobei die Zellgranulate aus den Metarhizium anisopliae Stämmen P 0001, entsprechend DSM 3884 oder P 0003, entsprechend DSM 3885 oder ihren Mutanten und Varianten gebildet werden.

7. Mittel gemäß den Ansprüchen 1 bis 6, erhältlich durch das Verfahren gemäß Anspruch 11.

8. Verwendung der Mittel gemäß den Ansprüchen 1 bis 7 zur Bekämpfung von Schädlingen oder zur Behandlung von Pflanzen.

9. Verwendung der Mittel gemäß den Ansprüchen 1 bis 7 zur Bekämpfung von Arthropoden und Nematoden, vorzugsweise von Insekten.

10. Verfahren zur Bekämpfung von Schädlingen, vorzugsweise von Arthropoden und Nematoden, insbesondere von Insekten, dadurch gekennzeichnet, daß man die Schädlingsbekämpfungsmittel gemäß den Ansprüchen 1 bis 7 auf die Schädlinge oder in ihren Lebensraum ausbringt.

11. Verfahren zur Herstellung von Schädlingsbekämpfungsmitteln und Pflanzenbehandlungsmitteln gemäß Anspruch 1, dadurch gekennzeichnet, daß man

(A) zur Initiation der Zellaggregation

a) im Falle von Mikroorganismenzellen mit einer im wesentlichen hydrophoben Zelloberfläche, nach Zugabe eines oder mehrerer Detergentien zu einer wäßrigen Aufschlämmung der in einer Vorkultur erhaltenen Mikroorganismen, die zur Schädlingsbekämpfung oder Pflanzenbehandlung geeignet sind, die Zellen suspendiert und die Zellsuspension in Wasser oder ein wäßriges Nährmedium einträgt, so daß eine Zellaggregation erfolgt oder

b) im Falle von Mikroorganismenzellen ohne eine im wesentlichen hydrophobe Zelloberfläche durch die Zugabe von Säuren oder Basen zu einer Aufschlämmung der in einer Vorkultur erhaltenen Mikroorganismen, die zur Schädlingsbekämpfung oder Pflanzenbehandlung geeignet sind, in Wasser oder einem wäßrigen Nährmedium den pH-Wert so einstellt, daß eine Zellaggregation erfolgt oder

c) eine Aufschlämmung bzw. Suspension der in einer Vorkultur erhaltenen Mikroorganismen, die zur Schädlingsbekämpfung oder Pflanzenbehandlung geeignet sind, in Wasser oder einem Nährmedium mit Flocculantien versetzt, so daß eine Zellaggregation erfolgt und anschließend.

(B) zur Bildung der Zellgranulate die erhaltenen Zellaggregationen in einem Nährmedium, welches gegebenenfalls komplexbildende Substanzen enthält, unter aeroben Bedingungen einer Fermentation unterwirft und die gebildeten Zellgranulate abtrennt und

(C) gegebenenfalls zur Bildung einer im wesentlichen auf der Oberfläche der Zellgranulate erhöhten Menge von Mikroorganismen-Dauerstadien, die abgetrennten Zellgranulate einer Inkubation unter den Bedingungen einer Oberflächenkultur unterwirft und

(D) die erhaltenen Zellgranulate, gegebenenfalls nach dem Hinzufügen von bzw. der Behandlung mit Nährstoffen, protektiv wirkenden Substanzen, antioxidativ wirkenden Stoffen und/oder die Rehydratation unterstützenden Stoffen, trocknet und gegebenenfalls mit weiteren Schädlingsbekämpfungsmitteln oder Pflanzenbehandlungsmitteln mischt.

12. Verwendung von zur Schädlingsbekämpfung oder Pflanzenbehandlung geeigneten Mikroorganismen, welche zur Myzelbildung fähig sind, zur Erzeugung der Mittel gemäß den Ansprüchen 1 bis 7.

13. Verwendung der Metarhizium anisopliae Stämme P 0001, entsprechend DSM 3884 und P 0003, entsprechend DSM 3885 sowie ihrer Mutanten und Varianten zur Erzeugung der Mittel gemäß den Ansprüchen 1 bis 7.

14. Metarhizium anisopliae Stämme P 0001, entsprechend DSM 3884 und P 0003, entsprechend DSM 3885 sowie ihre Mutanten, welche zur Herstellung der Schädlingsbekämpfungsmittel gemäß den Ansprüchen 1–7 geeignet sind.

## Revendications

1. Compositions destinées à la lutte contre des parasites et au traitement de plantes, caractérisées en ce qu'elles sont constituées de granulés cellulaires, dépourvus de support, de micro-organismes qui conviennent pour combattre des parasites ou pour traiter des plantes, ou contiennent au moins un produit granulé cellulaire sans support de ces micro-organismes, les granulés cellulaires étant formés à partir de champignons ou de bactéries qui sont capables de produire un mycélium et les granulés cellulaires ayant une forme principalement sphérique et présentant des diamètres de 0,05 à 2 mm.

2. Compositions suivant la revendication 1, dans lesquelles les granulés cellulaires contiennent des substances nutritives, des substances à action protectrice, des substances à action anti-oxydante et/ou des substances entretenant la réhydratation.

3. Compositions suivant les revendications 1 et 2, dans lesquelles les granulés cellulaires présentent principalement à leur surface une quantité élevée de stades résistants de micro-organismes.

4. Compositions suivant les revendications 1 à 3, dans lesquelles les granulés cellulaires sont formés à partir de champignons de la classe des deutéromycètes.

5. Compositions pesticides suivant les revendications 1 à 4, qui conviennent pour combattre des arthropodes et des nématodes, de préférence des insectes, les granulés cellulaires étant formés par des champignons du genre Metarhizium, de préférence l'espèce Metarhizium anisopliae.

6. Compositions pesticides suivant les revendications 1 à 5, dans lesquelles les granulés cellulaires sont formés à partir des souches P 0001, correspondant à DSM 3884, ou P 0003, correspondant à DSM 3885, de Metarhizium anisopliae ou leurs mutants et variants.

7. Compositions suivant les revendications 1 à 6, pouvant être obtenues par le procédé suivant la revendication 11.

8. Utilisation des compositions suivant les revendications 1 à 7 pour combattre des parasites ou pour traiter des plantes.

9. Utilisation des compositions suivant les revendications 1 à 7 pour combattre des arthropodes et des nématodes, de préférence des insectes.

10. Procédé pour combattre des parasites, de préférence des arthropodes et des nématodes, notamment des insectes, caractérisé en ce qu'on applique les compositions pesticides suivant les revendications 1 à 7 aux parasites ou à leur habitat.

11. Procédé de production de compositions pesticides et de compositions pour le traitement des plantes suivant la revendication 1, caractérisé en ce que

(A) pour le déclenchement de l'agrégation cellulaire

a) dans le cas de cellules de micro-organismes dont la surface est principalement hydrophobe, après addition d'un ou plusieurs détergents à une suspension aqueuse des micro-organismes, obtenus dans une culture préalable, qui conviennent pour la lutte contre les parasites ou pour le traitement de plantes, les cellules sont mises en suspension et la suspension cellulaire est versée dans l'eau ou dans un milieu nutritif aqueux, de telle manière qu'une agrégation cellulaire s'effectue, ou bien

b) dans le cas de cellules de micro-organismes ne présentant pas une surface essentiellement hydrophobe, on ajuste la valeur du pH par l'addition d'acides ou de bases à une suspension des micro-organismes, obtenus dans une culture préalable, qui conviennent pour la lutte contre les parasites ou pour le traitement de plantes, dans l'eau ou dans un milieu nutritif aqueux de manière qu'une agrégation des cellules s'effectue, ou bien

c) on ajoute des floculants à une suspension des micro-organismes, obtenus dans une culture préalable, qui conviennent pour la lutte contre des parasites ou le traitement de plantes, dans l'eau ou dans un milieu nutritif de manière qu'une agrégation des cellules s'effectue, après quoi

(B) pour la formation des granulés cellulaires les agrégats cellulaires obtenus sont exposés dans des conditions aérobies à une fermentation dans un milieu nutritif qui contient, le cas échéant, des substances formant des complexes et les granulés cellulaires formés sont séparés, et

(C) le cas échéant, en vue de la formation d'une quantité élevée de stades résistants de microorganismes principalement à la surface des granulés cellulaires, les granulés cellulaires séparés sont soumis à une incubation dans les conditions d'une culture en surface, et

(D) les granulés cellulaires obtenus, le cas échéant après addition de substances nutritives, de substances à action protectrice, de substances à action anti-oxydante et/ou de substances entretenant la réhydratation ou après traitement avec de telles substances, sont séchés et, le cas échéant, mélangés avec d'autres compositions pesticides ou d'autres compositions pour le traitement des plantes.

12. Utilisation de micro-organismes appropriés pour la lutte contre des parasites ou pour le traitement de plantes, qui sont capables de former un mycélium, pour l'obtention des compositions suivant les revendications 1 à 7.

13. Utilisation des souches P 0001, correspondant à DSM 3884, et P 0003, correspondant à DSM 3885, de Metarhizium anisopliae ainsi que de leurs mutants et variants pour l'obtention des compositions suivant les revendications 1 à 7.

14. Souches P 0001 correspondant à DSM 3884, et P 0003 correspondant à DSM 3885 de Metarhizium anisopliae, ainsi que leurs mutants, qui conviennent pour l'obtention de compositions pesticides suivant les revendications 1 à 7.

## Claims

1. Pesticides and plant treatment agents, characterized in that they consist of carrier-free cell granules of microorganisms which are suitable for combating pests or for treating plants, or at least contain carrier-free cell granules of such microorganisms, where the cell granules are formed by fungi or bacteria capable of forming a mycelium, and where the cell granules have an essentially spherical shape and diameters of from 0.05 to 2 mm.

2. Agents according to Claim 1, where the cell granules contain nutrients, substances with a protective action, substances with an antioxidant action and/or substances which aid rehydration.

3. Agents according to Claims 1 and 2, where the cell granules essentially have an increased amount of permanent stages of microorganisms on their surface.

4. Agents according to Claims 1 to 3, where the cell granules are formed from fungi of the class of the Deuteromycetes.

5. Pesticides according to Claims 1 to 4 which are suitable for combating arthropods and nematodes, preferably insects, where the cell granules are formed by fungi of the genus Metarhizium, preferably of the species Metarhizium anisopliae.

6. Pesticides according to Claims 1 to 5, wherein the cell granules are formed from the Metarhizium anisopliae strains P 0001, corresponding to DSM 3884, or P 0003, corresponding to DSM 3885, or their mutants and variants.

7. Agents according to Claims 1 to 6, obtainable by the process according to Claim 11.

8. Use of the agents according to Claims 1 to 7 for combating pests or for treating plants.

9. Use of the agents according to Claims 1 to 7 for combating arthropods and nematodes, preferably insects.

10. Method of combating pests, preferably arthropods and nematodes, in particular insects, characterized in that the pesticides according to Claims 1 to 7 are applied to the pests or their environment.

11. Process for the preparation of pesticides and plant treatment agents according to Claim 1, characterized in that

(A) to initiate the cell aggregation

a) in the case of microorganism cells with an essentially hydrophobic cell surface, after addition of one or more detergents to an aqueous slurry of the microorganisms which are suitable for combating pests or plant treatment and are kept in a preculture, the cells are suspended and the cell suspension is introduced into water or an aquenous nutrient medium, so that cell aggregation takes place, or

b) in the case of microorganism cells without an essentially hydrophobic cell surface, by addition of acids or bases to a slurry, in water or an aqueous nutrient medium, of the microorganisms which are suitable for combating pests or plant treatment and are kept in a preculture, the pH is adjusted in such a way that cell aggregation takes place, or

c) flocculants are added to a slurry or suspension, in water or a nutrient medium, of the microorganisms which are suitable for combating pests or plant treatment and are kept in a preculture, so that cell aggregation takes place, and subsequently,

(B) to form the cell granules the resulting cell aggregations are subjected to fermentation under aerobic conditions in a nutrient medium, which may contain complexing substances, and the cell granules formed are separated off, and

(C) if appropriate, to form an essentially increased amount of permanent stages of microorganisms on the surface of the cell granules, the cell granules separated off are subjected to incubation under the conditions of a surface culture, and

(D) the resulting cell granules, if appropriate after addition of or treatment with nutrients, substances with a protective action, substances with an antioxidant action and/or substances which aid rehydration, are dried and if appropriate mixed with other pesticides or plant treatment agents.

12. Use of microorganisms suitable for combating pests or treating plants and capable of forming a mycelium, for producing the agents according to Claims 1 to 7.

13. Use of the Metarhizium anisopliae strains P 0001, corresponding to DSM 3884, and P 0003, corresponding to DSM 3885, and of their mutants and variants, for producing the agents according to Claims 1 to 7.

14. Metarhizium anisopliae strains P 0001, corresponding to DSM 3884, and P 0003, corresponding to DSM 3885, and their mutants which are suitable for preparing the pesticides according to Claims 1 to 7.